# FASCICULE DE BREVET EUROPEEN

(11) **EP 3 060 673 B1**
(45) Date de publication et mention de la délivrance du brevet: **19.07.2017**
(21) Numéro de dépôt: 14806078.3
(22) Date de dépôt: 22.10.2014
(51) Int. Cl.: C12M 1/00, C12P 7/14, C12P 7/04, C12P 7/16

(54) **PROCEDE DE PRODUCTION D'UN PRODUIT ORGANIQUE A PARTIR D'UNE CHARGE DE MATIERE CARBONEE METTANT EN OEUVRE UNE GAZEIFICATION SUIVIE D'UNE FERMENTATION DU GAZ DE SYNTHESE**
VERFAHREN ZUR HERSTELLUNG EINES ORGANISCHEN PRODUKTS AUS EINEM KOHLENSTOFFHALTIGEN MATERIAL MITTELS VERGASUNG GEFOLGT DURCH FERMENTATION VON SYNTHESEGAS
METHOD FOR PRODUCING AN ORGANIC PRODUCT FROM A CARBON CONTAINING MATERIAL BY USING A GASIFICATION FOLLOWED BY FERMENTATION OF THE SYNTHESIS GAS

(30) Priorité: 24.10.2013 FR 1360398
(43) Date de publication de la demande: 31.08.2016
(73) Titulaire: Commissariat à l'Énergie Atomique et aux Énergies Alternatives, 75015 Paris (FR)
(72) Inventeur: SETIER, Pierre-Alexandre, F-38300 Bourgoin-Jallieu (FR); DELRUE, Florian, 84120 Pertuis (FR)
(74) Mandataire: Nony
(86) Numéro de dépôt international: PCT/IB2014/065547
(87) Numéro de publication internationale: WO 2015/059656

(56) Documents cités:
- EP-A2- 1 754 771
- WO-A1-2010/126382
- WO-A2-02/08438
- WO-A2-2010/056463
- US-A1- 2013 149 693
- MUNASINGHE P C ET AL: "Biomass-derived syngas fermentation into biofuels: Opportunities and challenges", BIORESOURCE TECHNOLOGY, ELSEVIER BV, GB, vol. 101, no. 13, 1 juillet 2010 (2010-07-01), pages 5013-5022, XP026986241, ISSN: 0960-8524 [extrait le 2010-03-27] cité dans la demande

## Description

### Domaine technique

La présente invention concerne un procédé de production d'un produit organique, en particulier d'un carburant à partir d'une charge de matière carbonée, mettant en oeuvre une gazéification de la charge de matière carbonée et une fermentation du gaz de synthèse produit par la gazéification au moyen de micro-organismes et d'eau.

Par « charge de matière carbonée », on entend toute matière contenant une quantité de carbone, en particulier toute matière carbonée de résidus, telle que charbon, pet coke, déchets organiques, déchets de matière plastique....

L'invention vise à améliorer le rendement d'un tel procédé de production d'un produit organique.

Une application préférée est la production d'éthanol ou de butanol à partir de résidus de matière plastique.

### Etat de la technique

La gazéification de la biomasse et du charbon est connue depuis longtemps. De manière générale, on peut la définir comme une transformation thermochimique de la biomasse ou du charbon par l'action de la chaleur en présence d'agent(s) gazéifiant(s). On cherche à générer, à l'issue de la gazéification, un mélange de gaz dit gaz de synthèse (« syngas » en anglais) qui comprend du monoxyde de carbone et de l'hydrogène (CO+H₂) entre autres.

Ainsi, la gazéification est une réaction endothermique entre la matière carbonée et l'(les) agent(s) gazéifiant(s) selon les réactions chimiques suivantes :

C + H₂O → CO + H₂ (1)

C + CO₂ → 2 CO (2)

Le gaz de synthèse produit est majoritairement constitué des gaz CO, CO₂, H₂.

Il contient également des composés minoritaires (NH₃, HCl, HF, HCN, H₂S, COS, métaux alcalins...) à l'état de vapeur provenant de la matière carbonée ou de l'(des) agent(s) gazéifiant(s). Enfin, pour des gazéifications à basse température (850°C), on obtient une part plus ou moins importante de composés hydrocarbonés sous forme gazeuse (goudrons, CH₄), résultant d'une oxydation incomplète de la matière carbonée.

Les réactions (1) et (2) doivent être favorisées. Il faut donc compenser l'énergie perdue lors de ces réactions endothermiques. L'apport de chaleur au réacteur de gazéification (gazéifieur) est soit direct (mode de fonctionnement dit autothermie), selon lequel on brûle une partie de la matière carbonée que l'on veut gazéifier, soit indirect (mode de fonctionnement dit allothermie), selon lequel on apporte une source de chaleur externe).

De conception et de construction simples, les procédés de gazéification mettant en oeuvre un réacteur à lit fixe ou à lit fluidisé produisent un gaz de relativement mauvaise qualité du fait d'une teneur en goudron relativement forte et d'un ratio H₂/CO obtenu relativement faible. Les réacteurs à lit fluidisé ont cependant l'avantage d'avoir un retour d'expérience important et de pouvoir être de dimensions compatibles avec une exploitation industrielle. Enfin, les réacteurs à flux entraîné sont mieux adaptés à la gazéification de gros volumes de matière carbonée, la puissance unitaire d'un gazéifieur de ce type pouvant être supérieure à 50 MWth. Du fait du temps de séjour très court de la charge de matière carbonée et des températures de fonctionnement très élevées, typiquement supérieures à 1400°C, le gaz de synthèse obtenu par les gazéifieurs à flux entraîné ne contient pratiquement pas de goudrons, ce qui a pour avantage d'éviter un étage supplémentaire de nettoyage du gaz de synthèse.

Un exemple de gazéification avantageux, est celui de la gazéification de la biomasse ligno-cellulosique qui permet de générer un gaz de synthèse et de produire en aval soit des carburants liquides, soit d'autres produits organiques. Cette gazéification se déroule en présence typiquement de vapeur d'eau vers 800-900°C pour des réacteurs à lit fluidisé. Classiquement, une telle gazéification convertit le carbone de la biomasse avec un gaz en sortie du gazéifieur de composition moyenne de 20-25 % en CO, 8-12 % en CH₄, 18-22 % en CO₂ et environ 38-42 % en H₂ et, des composés organiques C₂ à C₁₆ plus des composés inorganiques.

Il a déjà été proposé de réaliser la fermentation de gaz de synthèse au moyen de micro-organismes spécifiques qui sont capables de produire de façon prédominante un type de carburant dans des conditions de faibles température et pression.

Ces micro-organismes spécifiques appartiennent principalement à la famille des bactéries anaérobies du genre *Clostridium.* Par exemple, certaines d'entre elles ont pu être identifiées pour la conversion de gaz de synthèse en éthanol. La plupart de ces bactéries utilisent la voie métabolique, appelée le cycle de Wood-Ljundahl, ou la voie réductrice de l'acétyle-CoA.

Pour la conversion en éthanol, les réactions chimiques réalisées par les bactéries sont les suivantes :

6CO + 3H₂O -> C₂H₅OH + 4CO₂

6H₂ + 2CO₂ -> C₂H₅OH + 3H₂O.

Un milieu réactionnel spécifique riche en nutriments (minéraux et éléments traces tels que métaux et vitamines) est utilisé pour diriger et optimiser le flux énergétique dans la voie métabolique souhaitée, maximisant ainsi la production de carburant ou de produit organique souhaité.

Les avantages essentiels d'une synthèse par fermentation au moyen de micro-organismes par rapport à une synthèse par voie chimique peuvent être résumés comme suit :
- les conditions opératoires de température et de pression sont moindres,
- l'utilisation de micro-organismes en tant que catalyseurs biologiques permet de tolérer de manière plus importante les impuretés présentes au sein du gaz de synthèse, ainsi qu'une gamme plus large de ratios H₂/CO.

Les principales limitations de la fermentation d'un gaz de synthèse sont liées au transfert de masse entre les phases gaz et les phases liquides. Ces limitations sont à l'origine des productivités relativement modestes qui sont observées à l'heure actuelle.

Si ces technologies suscitent d'ores et déjà beaucoup d'intérêt pour la production d'éthanol voire même pour des mélanges éthanol/acétone/butanol, le potentiel de ces technologies de fermentation est bien supérieur si l'on considère les développements récents de l'ingénierie métabolique et/ou de la biologie synthétique.

Ainsi, il est d'ores et déjà reconnu que la fermentation de gaz de synthèse obtenu par gazéification de matières carbonées peut permettre, en fonction des microorganismes sélectionnés, d'obtenir différentes molécules telles que l'acétate, le formate, le butyrate, le n-butyrate, le lactate, le pyruvate, l'éthanol, le butanol ou l'acétone, et plus récemment, le 2,3-butanediol.

La concentration en produit organique synthétisé dans l'eau est faible, typiquement inférieure à 50 g/L pour l'éthanol. Les quantités d'eau nécessaires à la synthèse par fermentation sont donc très importantes.

A ce jour, la plupart de cette eau est réintroduite dans le fermenteur après distillation à la fois du média de culture de fermentation (eau et nutriments) et du (des) produit(s) organique(s) final(ux) que l'on cherche à obtenir.

Il s'avère en outre nécessaire de purger une partie de l'eau pour garder un taux en polluant restreint dans le milieu de fermentation.

De la même manière, seule une partie du média de culture débarrassée du (des) produit(s) organique(s) peut être recyclée dans le fermenteur. L'autre partie est considérée comme un déchet et doit être traitée comme tel.

Par ailleurs, une séparation physique entre les solides et les liquides, généralement par décantation et/ou filtration, est effectuée en sortie de fermenteur afin de récupérer les micro-organismes dont une partie peut le cas échéant être recyclée dans le fermenteur. Tout comme pour le média de culture, l'autre partie des micro-organismes est considérée comme un déchet et doit être traitée comme tel. En général, cette partie des micro-organismes formant des déchets est brûlée.

Par conséquent, la quantité de micro-organismes et d'eau à traiter dans un procédé de production connu de produit(s) organique(s) mettant en oeuvre une gazéification suivie d'une fermentation, est relativement importante et requière beaucoup d'énergie.

Il existe donc un besoin pour améliorer les procédés de production d'un carburant ou tout autre produit organique mettant en oeuvre une gazéification suivie d'une fermentation au moyen de micro-organismes et d'eau, notamment en vue d'en augmenter son rendement matière et de diminuer le coût en énergie de traitement de déchets constitués par lesdits micro-organismes et l'eau.

Le but général de l'invention est de répondre en partie à ce besoin.

Un but particulier est de proposer un procédé de production d'un carburant ou tout autre produit organique mettant en oeuvre une gazéification suivie d'une fermentation au moyen de micro-organismes et d'eau, avec un rendement matière augmenté, un coût moindre en énergie de traitement de déchets constitués par lesdits micro-organismes et l'eau diminué et en outre, d'investissement moindre.

### Exposé de l'invention

Pour ce faire, l'invention a pour objet un procédé de production d'un carburant, notamment un carburant liquide, ou d'un autre produit organique, à partir d'une charge de matière carbonée, comprenant les étapes suivantes :
a/ gazéification de la charge de matière carbonée dans un premier réacteur, dit gazéifieur,
b/ en aval de la gazéification, fermentation du gaz de synthèse produit selon l'étape a/, au moyen de micro-organismes, de l'eau et des nutriments dans un deuxième réacteur, dit fermenteur,
c/ récupération, en aval du fermenteur, des micro-organismes et de l'eau ;
d/ injection d'au moins une partie des micro-organismes et le cas échéant d'au moins une partie de l'eau récupérés en entrée du gazéifieur ;
   lors de l'étape de récupération c/ :
   - une étape c1/ de séparation entre les micro-organismes ayant servi à la fermentation et le mélange entre le produit organique issu de la fermentation et l'eau puis une distillation du mélange pour produire le produit organique final ;
   - une étape c2/ d'ajustement de la concentration en eau dans les micro-organismes récupérés par la séparation.

Ainsi, la présente invention propose un procédé qui peut recycler au moins une partie substantielle des déchets qu'il produit (micro-organismes et eau) directement dans le gazéifieur.

D'une part, ceci permet d'amener au gazéifieur de l'eau et du carbone supplémentaire qui est présent dans les micro-organismes et ainsi d'augmenter le rendement matière du procédé global.

D'autre part, ceci permet d'économiser le traitement de l'eau et des micro-organismes. Enfin, la présente invention permet d'ajuster à souhait le ratio entre l'eau et la matière carbonée en entrée de gazéifieur.

Le surcoût d'investissement lié à la mise en oeuvre de la présente invention dans une installation existante est faible car le cas échéant seule la mise en place de tuyauteries supplémentaire et d'un dispositif de dosage supplémentaire, adapté pour ajuster la concentration en eau dans les micro-organismes récupérés par la séparation, au moyen de l'eau récupérée de la distillation, est requise.

Pour réaliser l'étape c2/ d'ajustement, il est possible de soit introduire de l'eau dans le gazéifieur, soit introduire davantage de gaz afin de compenser l'eau en excès.

La première possibilité est préférée car elle est moins énergivore que la deuxième.

L'eau introduite dans le gazéifieur peut provenir en partie ou totalement de l'étape de distillation, ou elle peut provenir d'un circuit extérieur.

De préférence, pour éliminer toute présence d'impuretés, telles que le goudron et/ou le CO₂, entre l'étape a/ de gazéification et l'étape b/ de fermentation, on réalise une étape a1/ de nettoyage du gaz de synthèse produit selon l'étape a/.

Selon un mode de réalisation avantageux, les micro-organismes sont des micro-organismes anaérobies mésophiles, de préférence choisis parmi les espèces suivantes : *Clostridium jungdahlii, Clostridium carboxidovorans P7, Clostridium autoethanogenum, Eurobacterium limosum, Rhodospirillum rubrum, Peptostreptococcus productus, Acetobacterium woodii* ou *Butyribacterium methylotrophicum.* De manière plus générale, tous les micro-organismes cités dans les tableaux 2 et 3 de la publication [1] peuvent être avantageusement utilisés.

L'étape de séparation c1/ est de préférence réalisée par microfiltration ou par coagulation/floculation à l'aide d'un agent floculant à base de polymère organique, tel que la polyamine ou le polyacrylamide, suivie d'une décantation ou d'une flottation.

Selon cette variante, pour une concentration en micro-organismes récupérés par la séparation comprise entre 100 et 250 grammes/litre de concentrat, l'ajustement selon l'étape c2/ est de préférence réalisé de sorte à obtenir une concentration en micro-organismes à injecter dans le gazéifieur comprise entre 20 et 100 grammes/litre de solution.

La réaction de gazéification peut être avantageusement réalisée à des températures comprises 700 et 1600°C.

Selon une première variante, le gazéifieur est un réacteur de type à lit fluidisé, la réaction de gazéification étant réalisée à des températures comprises entre 800 et 950°C.

Selon une deuxième variante, le gazéifieur est un réacteur de type à flux entrainé, la réaction de gazéification étant réalisée à des températures comprises entre 1400 et 1600°C.

Le produit organique obtenu par le procédé selon l'invention peut être choisi parmi l'acétate, le formate, le butyrate, le n-butyrate, le lactate, le pyruvate, l'éthanol, le butanol ou l'acétone, et plus récemment, le 2,3-butanediol.

L'invention concerne également, sous un autre de ses aspects, une installation de production en continu d'un carburant, notamment un carburant liquide, ou d'un autre produit organique, à partir d'une charge de matière carbonée comportant :
- un gazéifieur,
- un fermenteur, en aval du gazéifieur, comportant en son sein des micro-organismes, de l'eau et des nutriments adaptés pour réaliser la fermentation du gaz de synthèse produit par le gazéifieur,
- des moyens de récupération d'au moins une partie des micro-organismes et de l'eau, en aval du fermenteur; les moyens de récupération étant reliés à l'entrée du gazéifieur de sorte à injecter au moins une partie des micro-organismes et le cas échéant d'au moins une partie de l'eau récupérés,
les moyens de récupération comprenant :
- un dispositif de séparation entre les micro-organismes et le mélange entre le produit organique issu de la fermentation et l'eau ayant servi à la fermentation au sein du fermenteur ;
- un dispositif de dosage, en aval du dispositif de séparation et d'un dispositif de distillation du mélange, adapté pour ajuster la concentration en eau dans les micro-organismes récupérés par la séparation.

Selon un mode de réalisation avantageux, le dispositif de séparation comprend au moins une membrane de filtration, dont la dimension des pores est adaptée à la taille des micro-organismes utilisés dans l'étape b/ de fermentation, de préférence comprise entre 0,1 et 10µm.

En ajustant l'efficacité de la filtration par la membrane de filtration, on peut fixer la teneur en eau dans le concentrat de micro-organismes. Si cela s'avère insuffisant, notamment en cas de besoin de plus d'eau pour la gazéification si la matière carbonée est très chargée en carbone par exemple, il est préféré d'ajouter de l'eau provenant de l'étape de distillation.

L'invention concerne enfin une application préférée du procédé qui vient d'être décrit ou de l'installation qui vient également d'être décrite pour produire de l'éthanol ou du butanol à partir de résidus de matière plastique.

### Description détaillée

D'autres avantages et caractéristiques de l'invention ressortiront mieux à la lecture de la description détaillée de l'invention faite à titre illustratif et non limitatif en référence aux figures suivantes parmi lesquelles :
- la figure 1 est une vue schématique du principe d'une installation de production d'éthanol à partir d'une charge de matière carbonée mettant en oeuvre en continu une gazéification suivie d'une fermentation au moyen de micro-organismes selon l'état de l'art;
- la figure 2 est une vue schématique du principe d'une installation de production d'éthanol à partir d'une charge de matière carbonée mettant en oeuvre en continu une gazéification suivie d'une fermentation au moyen de micro-organismes selon la présente invention.

Dans l'ensemble de la demande et en particulier dans la description qui va suivre les termes « entrée », « sortie » « amont », « aval », sont utilisés par référence avec la direction de transfert de la charge de matière carbonée et du gaz de synthèse dans l'installation de production d'un produit organique, tel que l'éthanol mettant en oeuvre le procédé selon l'invention.

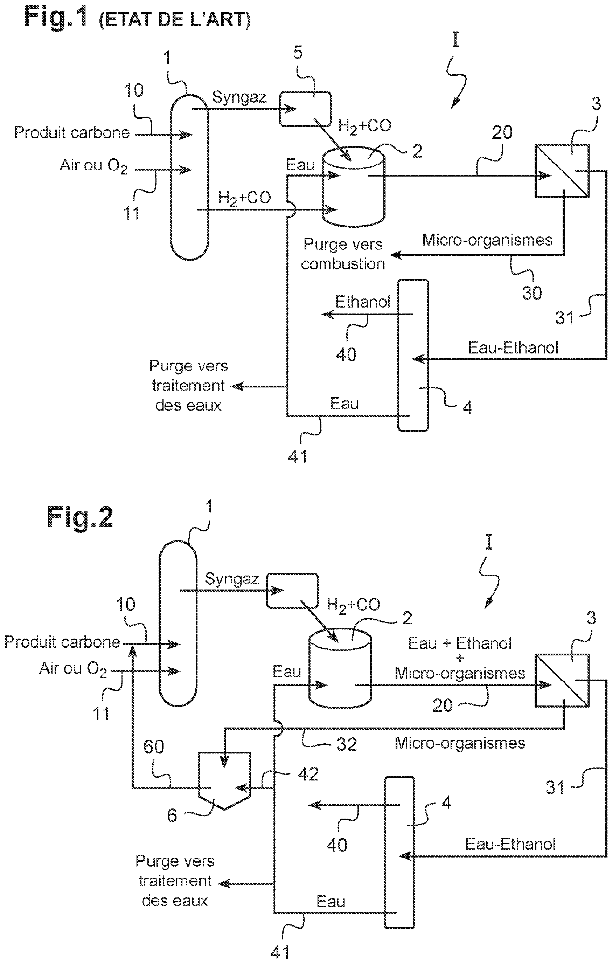

On précise que les légendes données en figures 1 et 2, et en particulier les réacteurs, et dispositifs indiquées ne le sont qu'à titre d'exemple non limitatif.

On précise que par souci de clarté les mêmes éléments dans une installation de production d'éthanol selon l'état de l'art et une installation de production d'éthanol selon l'invention sont désignés par les mêmes références.

Telle qu'illustrée en figure 1, l'installation I en continu met en oeuvre un procédé d'une gazéification d'une charge de matière carbonée selon l'état de l'art suivie d'une fermentation au moyen de micro-organismes pour la synthèse de l'éthanol.

Ainsi, l'installation I comporte tout d'abord un réacteur de gazéification ou gazéifieur 1, de type à lit fluidisé est alimenté en continu à son entrée 10 par exemple par des déchets de matière plastique depuis un réservoir de stockage non représenté. Le réacteur 1 à lit fluidisé peut fonctionner de préférence entre 800 et 950°C. Le réacteur 1 peut également être un réacteur de type à flux entraîné (EFR, acronyme anglais de « *Entrained flow reactor* ») fonctionnant de préférence à des températures comprises typiquement entre 1400-1600°C.

Le gazéifieur 1 est également alimenté à son entrée 11 par des agents gazéifiants, par exemple de l'air ou de l'oxygène.

A la sortie du réacteur de gazéification 1, un mélange brut de gaz de synthèse comprenant pour les espèces majoritaires du CO, CO₂, H₂O et H₂ est émis.

Le mélange de gaz de synthèse peut subir un nettoyage dans un dispositif 5 adapté afin d'extraire les polluants ou les gaz qui vont inhiber les micro-organismes du fermenteur 2.

Le gaz de synthèse nettoyé est alors envoyé vers un bio-réacteur 2 ou fermenteur mettant en oeuvre une fermentation au moyen de micro-organismes anaérobies mésophiles, de l'eau et des nutriments présents au sein du fermenteur 2, ce qui produit de l'éthanol selon les réactions:

6CO + 3H₂O -> C₂HₛOH + 4CO₂

6H₂ + 2CO₂ -> C₂H₅OH + 3H₂O.

L'eau, l'éthanol produit et les micro-organismes ayant servi à la fermentation sont alors extraits à la sortie du fermenteur 2 et envoyés par une ligne 20 vers un dispositif de séparation 3 solide/liquide comprenant au moins une membrane de microfiltration, i.e. dont les pores sont de dimension comprise entre 0,1 et 10µm.

Les solides constitués pas les micro-organismes ainsi séparés subissent alors une purge 30 pour être brûlés par combustion.

Le mélange liquide séparé et constitué de l'eau et de l'éthanol synthétisé est quant à lui envoyé par une ligne 31 dans un dispositif de distillation 4.

En sortie 40 du dispositif de distillation 4, l'éthanol distillé est récupéré. Il constitue le produit final que l'on peut utiliser.

En sortie 41 du dispositif de distillation 4, l'eau distillée est récupérée et pour une majeure partie recyclée en étant réinjectée en entrée du fermenteur 2, et pour la partie restante envoyée vers une unité de traitement des eaux non représentée.

Afin d'augmenter le rendement matière et de diminuer le coût en énergie de traitement de déchets constitués par lesdits micro-organismes et l'eau, dans l'installation selon l'état de l'art représentée en figure 1, les inventeurs ont pensé à réinjecter les micro-organismes et le cas échéant d'au moins une partie de l'eau récupérés en entrée 10 du gazéifieur 1.

Ainsi, comme montré en figure 2, dans une installation I conforme à l'invention, on prévoit tout d'abord en sortie du dispositif de séparation 3, une ligne 32 de récupération des micro-organismes récupérés qui alimente l'entrée d'un dispositif de dosage 6.

L'installation I conforme à l'invention comporte également une ligne 42 d'au moins une partie de l'eau issue de la distillation 4 qui vient également alimenter l'entrée du dispositif de dosage 6.

Ainsi, le dispositif de dosage 6, en aval du dispositif de séparation 3 et du dispositif de distillation 4 du mélange, permet d'ajuster la concentration en eau dans les micro-organismes récupérés par la séparation, au moyen de l'eau récupérée de la distillation.

La sortie du dispositif de dosage 6 est reliée à l'entrée 10 du gazéifieur 1 au moyen de la ligne 60 afin d'alimenter celui-ci en micro-organismes et de l'eau ainsi recyclés.

Ainsi, l'installation I selon l'invention représentée en figure 2 présente comparativement à l'installation selon l'état de l'art représentée en figure 1 les avantages suivants :
- retraitement des déchets de fermentation, à un coût énergétique faible;
- retraitement de l'eau issue de la fermentation dans l'étape de gazéification, cette eau se soustrait au besoin d'agent gazéifiant;
- augmentation du rendement matière ;
- ajustement à souhait du ratio eau/matière carbonée pour la gazéification 1.

Les inventeurs ont fait les premiers calculs de gain de rendement matière.

Les données sont les suivantes :
- ratio eau/charge de matière carbonée: de 0% à 50% ;
- concentration en micro-organismes lors de la synthèse 2 : de 0.5 à 2 g/L de milieu;
- concentration en micro-organismes pour la boucle de recyclage 32, 6, 60 : de 20 à 100 g/L.

Avec comme hypothèse un débit de charge de matière carbonée d'1 t/h, la production du produit organique, tel que l'éthanol, peut être avantageusement comprise entre 200 et 500 t/h avec un débit de micro-organismes à retraiter alors compris entre 10 et 20 kg/h.

Ainsi, on obtient une augmentation de rendement massique de l'ordre de 1,5 %.

Bien que décrite en référence exclusivement à l'éthanol l'installation de production peut servir à l'obtention d'un autre produit organique en un combustible ou un carburant, notamment un carburant liquide, ou un autre produit de synthèse, et peut être utilisée pour la production à partir de tout type de charges de matière carbonée (charbon, pet coke, déchets organiques, déchets de matière plastique...).

### Référence citée

[1]: « Biomass-derived syngas fermentation into biofuels: Opportunities and challenges » de P. C. Munasinghe, S. K. Khanal *Department of Molecular Biosciences and Bioengineering (MBBE), University of Hawai'i at Manoa, Agricultural Science, Bioresourcce (impact factor: 4.25). 07/2010; 101(13):5013-22. DOI:10.1016/j.biortech.2009.12.098.

## Revendications

1. Procédé de production d'un carburant, notamment un carburant liquide, ou d'un autre produit organique, à partir d'une charge de matière carbonée, comprenant les étapes suivantes :
a/ gazéification de la charge de matière carbonée dans un premier réacteur, dit gazéifieur (1),
b/ en aval de la gazéification, fermentation du gaz de synthèse produit selon l'étape a/, au moyen de micro-organismes, de l'eau et des nutriments dans un deuxième réacteur, dit fermenteur (2),
c/ récupération, en aval du fermenteur, des micro-organismes et de l'eau ;
d/ injection d'au moins une partie des micro-organismes et le cas échéant d'au moins une partie de l'eau récupérés en entrée (10) du gazéifieur.
et lors de l'étape de récupération c/ :
- une étape c1/ de séparation entre les micro-organismes ayant servi à la fermentation et le mélange entre le produit organique issu de la fermentation et l'eau puis une distillation du mélange pour produire le produit organique final,
- une étape c2/ d'ajustement de la concentration en eau dans les micro-organismes récupérés par la séparation.

2. Procédé de production selon la revendication 1, selon lequel entre l'étape a/ de gazéification et l'étape b/ de fermentation, on réalise une étape a1/ de nettoyage du gaz de synthèse produit selon l'étape a/.

3. Procédé de production selon l'une des revendications 1 ou 2, selon lequel les micro-organismes sont des micro-organismes anaérobies mésophiles, de préférence choisis parmi les espèces suivantes : *Clostridium jungdahlii, Clostridium carboxidovorans P7, Clostridium autoethanogenum, Eurobacterium limosum, Rhodospirillum rubrum, Peptostreptococcus productus, Acetobacterium woodii* ou *Butyribacterium methylotrophicum.*

4. Procédé de production selon l'une quelconque des revendications précédentes, l'étape de séparation c1/ est réalisée par microfiltration ou par coagulation/floculation à l'aide d'un agent floculant à base de polymère organique, tel que la polyamine ou le polyacrylamide, suivie d'une décantation ou d'une flottation.

5. Procédé de production selon l'une quelconque des revendications précédentes, selon lequel pour une concentration en micro-organismes récupérés par la séparation comprise entre 100 et 250 grammes/litre de concentrat, l'ajustement selon l'étape c2/ est réalisé de sorte à obtenir une concentration en micro-organismes à injecter dans le gazéifieur comprise entre 20 et 100 grammes/ litre de solution.

6. Procédé de production selon l'une quelconque des revendications précédentes, selon lequel la réaction de gazéification est réalisée à des températures comprises 700 et 1600°C.

7. Procédé de production selon l'une quelconque des revendications précédentes, selon lequel le gazéifieur est un réacteur de type à lit fluidisé, la réaction de gazéification étant réalisée à des températures comprises entre 800 et 950°C.

8. Procédé de production selon l'une quelconque des revendications 1 à 6, selon lequel le gazéifieur est un réacteur de type à flux entrainé, la réaction de gazéification étant réalisée à des températures comprises entre 1400 et 1600°C.

9. Procédé de production selon l'une des revendications précédentes, le produit organique obtenu étant choisi parmi l'acétate, le formate, le butyrate, l'éthanol, le butanol ou l'acétone, le 2,3-butanediol.

10. Installation (I) de production en continu d'un carburant, notamment un carburant liquide, ou d'un autre produit organique, à partir d'une charge de matière carbonée comportant :
- un gazéifieur (1),
- un fermenteur (2), en aval du gazéifieur, comportant en son sein des micro-organismes, de l'eau et des nutriments adaptés pour réaliser la fermentation du gaz de synthèse produit par le gazéifieur,
- des moyens de récupération (3, 6, 32, 42, 60) d'au moins une partie des micro-organismes et de l'eau, en aval du fermenteur; les moyens de récupération étant reliés à l'entrée (10) du gazéifieur de sorte à injecter au moins une partie des micro-organismes et le cas échéant d'au moins une partie de l'eau récupérés,
les moyens de récupération comprenant :
- un dispositif de séparation (3) entre les micro-organismes et le mélange entre le produit organique issu de la fermentation et l'eau ayant servi à la fermentation au sein du fermenteur,
- un dispositif de dosage (6), en aval du dispositif de séparation (3) et d'un dispositif de distillation (4) du mélange, adapté pour ajuster la concentration en eau dans les micro-organismes récupérés par la séparation.

11. Installation selon la revendication 10, le dispositif de séparation comprenant au moins une membrane de filtration, dont la dimension des pores est adaptée à la taille des micro-organismes utilisés dans l'étape b/ de fermentation, de préférence comprise entre 0,1 et 10µm.

12. Application du procédé selon l'une des revendications 1 à 9 ou de l'installation selon l'une des revendications 10 à 11 pour produire du méthanol ou de l'éthanol à partir de résidus de matière plastique.

## Patentansprüche

1. Verfahren zur Herstellung eines Treibstoffs, insbesondere eines flüssigen Treibstoffs oder eines anderen organischen Produkts aus einer Charge eines kohlenstoffhaltigen Materials, mit den folgenden Schritten:
a/ Vergasen der Charge des kohlenstoffhaltigen Materials in einem ersten Reaktor, der als Vergaser (1) bezeichnet wird,
b/ nach dem Vergasen, Fermentieren des in Schritt a/ erzeugten Synthesegases mit Hilfe von Mikroorganismen, Wasser und Nährstoffen in einem zweiten Reaktor, der als Fermenter (2) bezeichnet wird,
c/ Zurückgewinnen, nach dem Fermentieren, der Mikroorganismen und des Wassers;
d/ Injektion wenigstens eines Teils der Mikroorganismen und gegebenenfalls zumindest eines Teils des zurückgewonnenen Wassers am Einlass (10) des Vergasers,
und, während des Schrittes c/ der Zurückgewinnung:
- einen Schritt c1/ der Trennung der Mikroorganismen, die zu der Fermentation gedient haben, von dem Gemisch aus dem durch die Fermentation entstandenen Produkt und dem Wassers und der anschließenden Destillation des Gemisches, um das organische Endprodukt zu erhalten,
- einen Schritt c2/ der Einstellung der Konzentration des Wassers in den durch die Trennung zurückgewonnenen Mikroorganismen.

2. Verfahren nach Anspruch 1, bei dem zwischen dem Schritt a/ der Vergasung und dem Schritt b/ der Fermentation ein Schritt a1/ der Reinigung des in Schritt a/ erzeugten Synthesegases ausgeführt wird.

3. Verfahren nach Anspruch 1 oder 2, bei dem die Mikroorganismen mesophile anaerobe Mikroorganismen sind, vorzugsweise ausgewählt unter den folgenden Arten: Clostridium jungdahlii, Clostridium carboxidovorans P7, Clostridium autoethanogenum, Eurobacterium limosum, Rhodospirillum rubrum, Peptostreptococcus productus, Acetobacterium woodii oder Butyribacterium methylotrophicum.

4. Verfahren nach einem der vorstehenden Ansprüche, bei dem der Schritt c1/ durch Mikrofiltration oder durch Koagulation/Flockung mit Hilfe eines Flockungsmittels auf der Basis eines organischen Polymers wie etwa Polyamin oder Polyacrylamid ausgeführt wird, gefolgt von einer Dekantation oder Flotation.

5. Verfahren nach einem der vorstehenden Ansprüche, bei dem, für eine Konzentration der Mikroorganismen, die durch die Trennung zurückgewonnen wurden, zwischen 100 und 250 g pro liter Konzentrat, die Einstellung gemäß Schritt c2/ derart ausgeführt wird, dass man eine Konzentration der in den Vergaser zu injizierenden Mikroorganismen zwischen 20 und 100 g pro liter Lösung erhält.

6. Verfahren nach einem der vorstehenden Ansprüche, bei dem die Vergasungsreaktion bei Temperaturen zwischen 700 und 1600° C ausgeführt wird.

7. Verfahren nach einem der vorstehenden Ansprüche, bei dem der Vergaser ein Fließbettreaktor ist und die Vergasungsreaktion bei Temperaturen zwischen 800 und 950° C ausgeführt wird.

8. Verfahren nach einem der Ansprüche 1 bis 6, bei dem der Vergaser ein Vergaser mit angetriebener Strömung ist und die Vergasungsreaktion bei Temperaturen zwischen 1400 und 1600° C ausgeführt wird.

9. Verfahren nach einem der vorstehenden Ansprüche, bei dem das erhaltene organische Produkt ausgewählt ist unter Azetat, Formiat, Butyrat, Ethanol, Butanol oder Azeton, 2,3-Butandiol.

10. Anlage (I) zur kontinuierlichen Herstellung eines Treibstoffs, insbesondere eines flüssigen Treibstoffs oder eines anderen organischen Produkts, aus einer Charge kohlenstoffhaltigen Materials, mit:
- einem Vergaser (1),
- einem Fermenter (2), stromabwärts des Vergasers, der im Inneren Mikroorganismen, Wasser und Nährstoffe enthält, geeignet für die Fermentation des von dem Vergaser erzeugten Synthesegases,
- Mitteln (3, 6, 32, 42, 60) zur Rückgewinnung wenigstens eines Teils der Mikroorganismen und des Wassers, stromabwärts des Fermenters, wobei die Rückgewinnungsmittel mit dem Einlass (10) des Vergasers verbunden sind, derart, dass wenigstens ein Teil der Mikroorganismen und gegebenenfalls wenigstens ein Teil des zurückgewonnenen Wassers injiziert wird,
wobei diese Rückgewinnungsmittel umfassen:
- eine Trenneinrichtung (3) zur Trennung der Mikroorganismen von dem Gemisch aus dem durch die Fermentation erhaltenen organischen Produkt und dem Wasser, das zur Fermentation in dem Fermenter gedient hat,
- eine Dosiereinrichtung (6), stromabwärts der Trenneinrichtung (3), und eine Destillationseinrichtung (4) zur Destillation des Gemisches, zur Einstellung der Konzentration des Wassers in den durch die Trennung zurückgewonnenen Mikroorganismen.

11. Anlage nach Anspruch 10, bei der die Trenneinrichtung wenigstens eine Filtrationsmembran aufweist, deren Porengröße an die Größe der in dem Fermentationsschritt d/ verwendeten Mikroorganismen angepasst ist, vorzugsweise zwischen 0,1 und 10 µm.

12. Anwendung des Verfahrens nach einem der Ansprüche 1 bis 9 oder der Anlage nach Anspruch 10 oder 11 für die Herstellung von Methanol oder Ethanol aus Kunststoffresten.

## Claims

1. A process for the production of a fuel, in particular a liquid fuel, or of another organic product, from a carbon-based matter feedstock, comprising the following stages:
a/ gasification of the carbon-based matter feedstock in a first reactor, referred to as gasifier (1),
b/ downstream of the gasification, fermentation of the synthesis gas produced according to stage a/ using microorganisms, water and nutrients in a second reactor, referred to as fermenter (2),
c/ recovery, downstream of the fermenter, of the microorganisms and water,
d/ injection of at least a portion of the recovered microorganisms and, if appropriate, of at least a portion of the recovered water at the inlet (10) of the gasifier,
and, during the recovery stage c/:
- a stage c1/ of separation between the microorganisms which have been used for the fermentation and the mixture between the organic product resulting from the fermentation and the water, followed by a distillation of the mixture in order to produce the final organic product,
- a stage c2/ of adjustment of the concentration of water in the microorganisms recovered by the separation.

2. The production process as claimed in claim 1, according to which, between the gasification stage a/ and the fermentation stage b/, a stage a1/ of cleaning the synthesis gas produced according to stage a/ is carried out.

3. The production process as claimed in either of claims 1 and 2, according to which the microorganisms are mesophilic anaerobic microorganisms, preferably chosen from the following species: *Clostridium jungdahlii, Clostridium carboxidovorans P7, Clostridium autoethanogenum, Eurobacterium limosum, Rhodospirillum rubrum, Peptostreptococcus productus*, *Acetobacterium woodii* or *Butyribacterium methylotrophicum.*

4. The production process as claimed in any one of the preceding claims, the separation stage c1/ is carried out by microfiltration or by coagulation/flocculation using a flocculating agent based on an organic polymer, such as polyamine or polyacrylamide, followed by separation by settling or by floatation.

5. The production process as claimed in any one of the preceding claims, according to which, for a concentration of microorganisms recovered by the separation of between 100 and 250 grams/liter of concentrate, the adjustment according to stage c2/ is carried out so to obtain a concentration of microorganisms to be injected into the gasifier of between 20 and 100 grams/liter of solution.

6. The production process as claimed in any one of the preceding claims, according to which the gasification reaction is carried out at temperatures of 70 and 1600°C.

7. The production process as claimed in any one of the preceding claims, according to which the gasifier is a reactor of fluidized bed type, the gasification reaction being carried out at temperatures of between 800 and 950°C.

8. The production process as claimed in any one of claims 1 to 6, according to which the gasifier is a reactor of entrained-flow type, the gasification reaction being carried out at temperatures of between 1400 and 1600°C.

9. The production process as claimed in one of the preceding claims, the organic product obtained being chosen from acetate, formate, butyrate, ethanol, butanol or acetone, 2,3-butanediol.

10. A plant (I) for the continuous production of a fuel, in particular a liquid fuel, or of another organic product from a carbon-based matter feedstock comprising:
- a gasifier (1),
- a fermenter (2), downstream of the gasifier, comprising within it microorganisms, water and nutrients appropriate for carrying out the fermentation of the synthesis gas produced by the gasifier,
- means for recovery (3, 6, 32, 42, 60) of at least a portion of the microorganisms and water, downstream of the fermenter; the recovery means being connected to the inlet (10) of the gasifier so as to inject at least a portion of the recovered microorganisms and, if appropriate, at least a portion of the recovered water,
the recovery means comprising:
- a device (3) for separation between the microorganisms and the mixture between the organic product resulting from the fermentation and the water which has been used for the fermentation within the fermenter,
- a metering device (6), downstream of the separation device (3) and of a device (4) for distillation of the mixture, appropriate for adjusting the concentration of water in the microorganisms recovered by the separation.

11. The plant as claimed in claim 10, the separation device comprising at least one filtration membrane, the size of the pores of which is suited to the size of the microorganisms used in the fermentation stage b/, preferably of between 0.1 and 10 µm.

12. The application of the process as claimed in one of claims 1 to 9 or of the plant as claimed in either of claims 10 and 11 for producing methanol or ethanol from plastic waste products.
